# EUROPEAN PATENT APPLICATION

(11) **EP 4 148 035 A1**
(43) Date of publication of application: **15.03.2023**
(21) Application number: 21196699.9
(22) Date of filing: 14.09.2021
(51) Int. Cl.: C07C 29/151, C07C 31/04

(54) **METHANOL SYNTHESIS BASED ON A MATHEMATICAL MODEL**

(71) Applicant: Air Liquide Societe Anonyme pour l'Etude et L'Exploitation des procedes Georges Claude, 75321 Paris Cedex 07 (FR)
(72) Inventor: DO, Nga Thi Quynh, 60388 Frankfurt am Main (DE); ÖRS, Evrim, 60388 Frankfurt am Main (DE); HAAG, Stéphane, 60388 Frankfurt am Main (DE)
(74) Representative: Schwenderling, Jens

(57) **Abstract**

Method for synthesizing methanol (1) from raw material (2) using a set of process parameters (3), wherein a mathematical model (4) is provided that is configured for calculating, based on the process parameters (3) and on at least one material property (5) of the raw material (2), an expected amount (6) of at least one by-product (7) of the synthesizing of the methanol (1), wherein the following steps are performed at least once in the stated order:
a) applying the mathematical model (4) to current values for the process parameters (3),
b) changing a respective set point for at least one of the process parameters (3) such that based on the mathematical model (4) a lower amount of the by-products (7) is expected under condition that at least a predetermined amount of the methanol can be synthesized.

## Description

The invention is directed to methods and a device for synthesizing methanol.

It is known to synthesize methanol from raw material such as hydrogen, carbon monoxide and carbon dioxide. Besides the desired methanol, also by-products can be formed in the synthesis process. The by-products can impair the efficiency of the synthesis process. Also, the by-products can cause problems once the amount of the by-products exceed what the used equipment is capable of handling.

The amount of the generated by-products depends in particular on the properties of the raw material. Known methods for producing methanol are designed for raw material of uniform properties as is the case if syngas is used as raw material. That is, with known methods the production of by-products can be kept in acceptable limits provided that the properties of the raw material stay within narrow limits. However, if these known methods are applied to raw material having fluctuating properties, the amount of the by-products can increase significantly and exceed the acceptable limits. That is, known methods are only applicable to high-quality raw material, which is expensive and difficult to obtain.

The object of the invention is to improve the prior art such that methanol can be synthesized with fewer requirements regarding the raw material. In particular, the amount of the generated by-products is supposed to be reduced.

The object is solved with the methods and the device according to the independent claims. Advantageous refinements are presented in the dependent claims. The features described in the claims and in the description can be combined with each other in any technologically reasonable manner.

According to the invention a method for synthesizing methanol from raw material using a set of process parameters is presented, wherein a mathematical model is provided that is configured for calculating, based on the process parameters and on at least one material property of the raw material, an expected amount of at least one by-product of the synthesizing of the methanol, wherein the following steps are performed at least once in the stated order:
a) applying the mathematical model to current values for the process parameters,
b) changing a respective set point for at least one of the process parameters such that based on the mathematical model a lower amount of the by-products is expected under condition that at least a predetermined amount of the methanol can be synthesized.

With the described method methanol, CH₄O, is produced. That is, the method comprises the actual step of synthesizing the methanol. The methanol can be synthesized from raw material such as hydrogen, carbon monoxide and carbon dioxide, for example using a copper catalyst. The synthesizing is performed using a set of process parameters. A process parameter can be used to control the process or to monitor the process. A process parameter can be any physical quantity involved in the synthesis process that can be influenced either directly, for example by adjusting the setting of a voltage source or a heating installation, or indirectly, for example by adjusting one or more other process parameters. For example, the set of process parameters can comprise voltage, current, temperature and/or pressure parameters. Each process parameter has a respective value that can vary over time. For example, a temperature at a certain location can be a process parameter, which can have a value of 25 °C at a certain first point of time and a value of 30 °C at a certain second point of time. The process is can be controlled such that a respective value for one or more of the process parameters approaches a respective set point.

In the method a mathematical model is used that is configured for calculating, based on the process parameters and on at least one material property of the raw material, an expected amount of at least one by-product of the synthesizing of the methanol. The mathematical model is used to predict how much of the at least one by-product is expected to be obtained based on the process parameters and on the at least one material property of the raw material. The mathematical model has a respective input for each of the process parameters and for each of the at least one material property. As an output, the mathematical model provides the expected amount of the at least one by-product. In case there is more than one by-product, the amount of the at least one by-product is preferably a sum of the respective amounts of the individual by-products. This applies wherever an amount of the at least one by-product is addressed, in particular to the expected amount of the at least one by-product as well as to an actually generated amount of the at least one by-product.

The output can be calculated for any combination of values for the process parameters and for the at least one material property. The input values can be based on measurements, assumptions, predictions or calculations. The obtained result consequently reflects how much of the at least one by-product is actually obtained or would be obtained in case the values for the process parameters and for the at least one material property are in fact as assumed, predicted or calculated, respectively. Hence, the mathematical model can be used to predict the amount of the at least one by-product for a certain combination of values for the process parameters and for the at least one material property even before the process parameters are actually set to these values and/or before raw material having these properties is used for the synthesis.

Hence, it is possible to control the process parameters according to steps a) and b) that are performed at least once in the stated order. In step a) the mathematical model is applied to the current values for the process parameters. Preferably, therein respective values for the at least one material property of currently used raw material are used. That is, in step a) it can be predicted how much of the at least one by-product is expected to be obtained in a current situation. Step a) can be performed independently of the actual synthesizing of the methanol. That is, it is not necessary that methanol is actually synthesized under the conditions used for step a). In case methanol is actually synthesized under the conditions used for step a), it is still reasonable to perform step a) rather than merely performing a measurement of the actual amount of the produced by-product. In particular, it is possible that the results of step a) are available earlier than measurement results.

With step a) a reference value for the amount of the by-products can be determined. Since the method aims at reducing the amount of the generated by-products, step b) is performed. Therein, a respective set point for at least one of the process parameters is changed such that based on the mathematical model a lower amount of the by-products is expected. Therein, the term "lower" refers to a comparison with the result obtained in step a). Step b) is performed under the condition that at least a predetermined amount of the methanol can be synthesized from the raw material using the values for the process parameters obtained by step b). This boundary condition ensures that the amount of the generated at least one by-product is not merely reduced by reducing the methanol output.

For example, step b) can be performed such that the mathematical model is applied to one or several sets of values for the process parameters, wherein for each set it is determined whether or not the result is lower than the result of step a). Preferably, the process parameters are changed to the values for which the mathematical model yields the lowest result. However, it is not necessary that the mathematical model is actually applied to the values to which the process parameters are changed. The described advantages would still be achieved, for example, even if values are used that are slightly different from the best set of parameters to which the mathematical model has been applied. Also, the mathematical model can be used to obtain the optimal set of values for the process parameters in an analytical manner. Therein, it might not be necessary to actually calculate the amount of the at least one by-product for the resulting optimal set of values for the process parameters.

In a preferred embodiment of the method steps a) and b) are performed repeatedly in an alternating manner.

With step b) the expected amount of the by-product is reduced. However, this refers to a comparison to the result of the corresponding step a). Hence, the expected amount of the at least one by-product does not necessarily decrease continuously. In particular, the raw material can change between iterations of steps a) and b). The described method even focuses on raw material having fluctuating material properties. For example, a first iteration of steps a) and b) might result in optimal values for the process parameters. Using these values, a minimal amount of the at least one by-product is generated. However, once the material properties of the raw material change, a significantly larger amount of the at least one by-product might be generated. If now steps a) and b) are performed again, a reduced amount of the at least one by-product may be achieved that is higher than the previously achieved minimal amount.

In a further preferred embodiment of the method steps a) and b) are performed while the methanol is synthesized continuously.

In the present embodiment the mathematical model can be used to influence the process while it is running. Thereby, the process parameters can be adapted continuously so as to compensate for fluctuations in the raw material. The synthesis of the methanol can begin prior to or simultaneously with the first iteration of step a).

In a further preferred embodiment of the method steps a) and b) constitute a real time control of the process parameters.

In the present embodiment the described method can be used for real time optimization of the process parameters. That is, except for unavoidable processing delays the set points for the process parameters are changed as soon as the conditions change.

In a further preferred embodiment of the method step b) is performed such that the expected amount of the at least one by-product is minimized.

In this embodiment the expected amount of the at least one by-product is not only reduced compared to the current process parameters, but minimized. To this end, optimal values for the process parameters can be found.

In a further preferred embodiment of the method the at least one by-product is/are chosen from the following: alcohols, ketones, paraffins, esters, ethers.

Each by-product can be an alcohol, a ketone, a paraffin, an ester or an ether. In case there are multiple by-products, it is possible that, for example, a first by-product is a first alcohol and a second by-product is a second alcohol. The same applies analogously to ketones, paraffins, esters and ethers.

In a further preferred embodiment of the method in step a) for at least one of the process parameters a respective current measurement result is input into the mathematical model as the current value.

In this embodiment the actual current values for the process parameters can be used in the mathematical model. Preferably, in step a) for all the process parameters a respective current measurement result is input into the mathematical model as the current value.

In a further preferred embodiment of the method in step a) for at least one of the process parameters a respective current set point is input into the mathematical model as the current value.

In this embodiment the actual current values for the process parameters are approximated in that the respective set points are used. Ideally, the actual current values for the process parameters are identical to the respective set points. In such a case the present embodiment and the previous embodiment would provide the same result. Preferably, in step a) for all the process parameters a respective current set point is input into the mathematical model as the current value.

The presently described embodiment can be combined with the previously described embodiment in that in step a) for one or more of the process parameters a respective current measurement result is input into the mathematical model as the current value and for one or more other of the process parameters a respective current set point is input into the mathematical model as the current value.

In a further preferred embodiment the method further comprises measuring an actual amount of the generated at least one by-product and comparing the result with a corresponding prediction obtained according to step a).

Applying the mathematical model to the current values for the process parameters and to the current values for the at least one material property of the raw material yields a prediction of how much of the at least one by-product would be generated under respective conditions. However, the actual amount of the at least one by-product may differ from this prediction, for example due to inaccuracies in the mathematical model. In the present embodiment the deviation of the prediction from the actual result can be determined. Preferably, in case the prediction obtained in step a) deviates from the actual amount of the generated at least one by-product by more than a predetermined threshold value, the mathematical model is updated.

As a further aspect of the invention a method for synthesizing methanol from raw material using a set of process parameters is presented, wherein a mathematical model is provided that is configured for calculating, based on the process parameters and on at least one material property of the raw material, an expected amount of at least one by-product of the synthesizing of the methanol, wherein, with the mathematical model, a set of optimized values for the process parameters is determined by minimizing the expected amount of the at least one by-product under condition that at least a predetermined amount of the methanol can be synthesized, and wherein the methanol is synthesized from the raw material using values for the process parameters that each deviate by less than 20 % from the respective optimized value.

The advantages and features of the previously described methods are transferrable to the presently described method, and vice versa.

In the previously described method the mathematical model was used to reduce the expected amount of the at least one by-product compared to an initial set of values for the process parameters. The presently described method provides an alternative solution for obtaining a low amount of the at least one by-product. In both methods the same mathematical model can be used. Also, both methods share the general concept that the mathematical model is used to improve the values for the process parameters in order to reduce the amount of the generated at least one by-product.

In the presently described method, the process parameters are optimized such that the expected amount of the at least one by-product is minimized. The minimization is performed under the condition that at least a predetermined amount of the methanol can be synthesized from the raw material using the optimized values for the process parameters. This boundary condition ensures that the amount of the generated at least one by-product is not merely reduced by reducing the methanol output.

In the presently described method the methanol is preferably synthesized from the raw material using the optimized values for the process parameters. In this case the described advantages can be achieved to a full extent. However, a slight deviation from the optimized values would still result in an advantage. Hence, it is defined that the methanol is synthesized from the raw material using values for the process parameters that each deviate by less than 20 % from the respective optimized value. It was found that with a deviation of less than 20 % from the optimized values the described advantages can still be achieved to a substantial extent.

In a preferred embodiment of the method the methanol is synthesized from the raw material using values for the process parameters that each deviate by less than 10 % from the respective optimized value.

In a further preferred embodiment of the method a respective set of optimized values for the process parameters is determined at different points of time, wherein the methanol is synthesized from the raw material using values for the process parameters that each deviate by less than 20 %, preferably less than 10 %, from the respective optimized value of the most recent set of optimized values.

In this embodiment the optimization is not only performed once, but multiple times. This way, fluctuating material properties of the raw material can be handled particularly well.

As a further aspect of the invention a device for synthesizing methanol is presented. The device comprises a reactor and a control unit configured for performing one of the described methods.

The advantages and features of the described methods are transferrable to the device, and vice versa.

In the following the invention will be described with respect to the figure. The figure shows a preferred embodiment, to which the invention is not limited. The figure and the dimensions shown therein are only schematic. The figure shows:

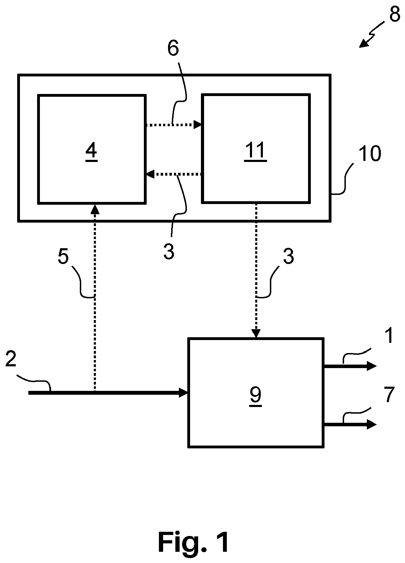

- Fig. 1:: a device for synthesizing methanol according to the invention.

Fig. 1 shows a device 8 for synthesizing methanol 1. The device 8 comprises a reactor 9, in which the methanol 1 can be synthesized from raw material 2. Besides the methanol 1, also at least one by-product 7 can be generated. Further, the device 8 comprises a control unit 10, which is configured for performing one of the following methods.

Both methods are configured for synthesizing the methanol 1 from the raw material 2 using a set of process parameters 3. In the control unit 10 a mathematical model 4 is implemented that is configured for calculating, based on the process parameters 3 and on at least one material property 5 of the raw material 2, an expected amount 6 of the at least one by-product 7 of the synthesizing of the methanol 1. Besides the mathematical model 4, the control unit 10 also comprises a processor 11.

In a first method the following steps are performed at least once in the stated order:
a) applying the mathematical model 4 to current values for the process parameters 3,
b) changing a respective set point for at least one of the process parameters 3 such that based on the mathematical model 4 a lower amount of the by-products 7 is expected under condition that at least a predetermined amount of the methanol 1 can be synthesized.

In a second method, with the mathematical model 4, a set of optimized values for the process parameters 3 is determined by minimizing the expected amount 6 of the at least one by-product 7 under condition that at least a predetermined amount of the methanol 1 can be synthesized, and wherein the methanol 1 is synthesized from the raw material 2 using values for the process parameters 3 that each deviate by less than 20 % from the respective optimized value.

### List of reference numerals

- 1: methanol
- 2: raw material
- 3: process parameter
- 4: mathematical model
- 5: material property
- 6: expected amount
- 7: by-product
- 8: device
- 9: reactor
- 10: control unit
- 11: processor

## Claims

1. Method for synthesizing methanol (1) from raw material (2) using a set of process parameters (3), wherein a mathematical model (4) is provided that is configured for calculating, based on the process parameters (3) and on at least one material property (5) of the raw material (2), an expected amount (6) of at least one by-product (7) of the synthesizing of the methanol (1), wherein the following steps are performed at least once in the stated order:
a) applying the mathematical model (4) to current values for the process parameters (3),
b) changing a respective set point for at least one of the process parameters (3) such that based on the mathematical model (4) a lower amount of the by-products (7) is expected under condition that at least a predetermined amount of the methanol (1) can be synthesized.

2. Method according to claim 1, wherein steps a) and b) are performed repeatedly in an alternating manner.

3. Method according to one of the preceding claims, wherein steps a) and b) are performed while the methanol (1) is synthesized continuously.

4. Method according to one of the preceding claims, wherein steps a) and b) constitute a real time control of the process parameters (3).

5. Method according to one of the preceding claims, wherein step b) is performed such that the expected amount (6) of the at least one by-product (7) is minimized.

6. Method according to one of the preceding claims, wherein the at least one by-product (7) is/are chosen from the following: alcohols, ketones, paraffins, esters, ethers.

7. Method according to one of the preceding claims, wherein in step a) for at least one of the process parameters (3) a respective current measurement result is input into the mathematical model (4) as the current value.

8. Method according to one of the preceding claims, wherein in step a) for at least one of the process parameters (3) a respective current set point is input into the mathematical model (4) as the current value.

9. Method according to one of the preceding claims, further comprising measuring an actual amount of the generated at least one by-product (7) and comparing the result with a corresponding prediction obtained according to step a).

10. Method for synthesizing methanol (1) from raw material (2) using a set of process parameters (3), wherein a mathematical model (4) is provided that is configured for calculating, based on the process parameters (3) and on at least one material property (5) of the raw material (2), an expected amount (6) of at least one by-product (7) of the synthesizing of the methanol (1), wherein, with the mathematical model (4), a set of optimized values for the process parameters (3) is determined by minimizing the expected amount (6) of the at least one by-product (7) under condition that at least a predetermined amount of the methanol (1) can be synthesized, and wherein the methanol (1) is synthesized from the raw material (2) using values for the process parameters (3) that each deviate by less than 20 % from the respective optimized value.

11. Method according to claim 10, wherein the methanol (1) is synthesized from the raw material (2) using values for the process parameters (3) that each deviate by less than 10 % from of the respective optimized value.

12. Method according to claim 10 or 11, wherein a respective set of optimized values for the process parameters (3) is determined at different points of time, and wherein the methanol (1) is synthesized from the raw material (2) using values for the process parameters (3) that each deviate by less than 20 % from the respective optimized value of the most recent set of optimized values.

13. Device (8) for synthesizing methanol (1) comprising a reactor (9) and a control unit (10) configured for performing a method according to one of the preceding claims.
